# EUROPÄISCHE PATENTSCHRIFT

(11) **EP 1 219 304 B1**
(45) Veröffentlichungstag und Bekanntmachung des Hinweises auf die Patenterteilung: **20.10.2004**
(21) Anmeldenummer: 01130803.8
(22) Anmeldetag: 24.12.2001
(51) Int. Cl.: A61K 31/196, A61K 47/18, A61K 9/08

(54) **Stabile Infusionslösung von Diclofenac-Salzen, deren Herstellung und Verwendung**
Stable parenteral solution containing diclofenac salts, their preparation and use therof
Solution parenterale stable d' un sel de diclofenac, sa fabrication et son utilisation

(30) Priorität: 28.12.2000 DE 10065110
(43) Veröffentlichungstag der Anmeldung: 03.07.2002
(73) Patentinhaber: Fresenius Kabi Austria GmbH, 8055 Graz (AT)
(72) Erfinder: Pomper, Herbert, Dr., 8075 Hart bei Graz (AT); Feichtinger, Norbert, Dr., 8053 Graz (AT); Eggenreich, Udo, Dr., 8020 Graz (AT); Haszonits, Oskar, Dr., 8010 Kainbach bei Graz (AT); Wurm, Anneliese, Dr., 8010 Graz (AT)
(74) Vertreter: Luderschmidt, Schüler & Partner GbR

(56) Entgegenhaltungen:
- US-A- 5 389 681
- UITZ E, AGLAS F, WURM A, RAINER F: WIENER MEDIZINISCHE WOCHENSCHRIFT, Bd. 148, Nr. 7, 1998, Seiten 179-182, XP002197543

## Beschreibung

Die vorliegende Erfindung betrifft eine lagerstabile Infusionslösung von Diclofenac-Salzen in therapieoptimierter Konzentration in wäßriger Lösung ohne jeglichen Zusatz an Lösungsvermittlern oder organischen Lösungsmitteln, die zur Behandlung von insbesondere akuten Schmerzzuständen vorgesehen ist und sich durch sehr gute Verträglichkeit auszeichnet.

Diclofenac-Salze, insbesondere Diclofenac-Natrium (2-(2',6'-Dichloranilino)-phenylessigsäure-Na-Salz) 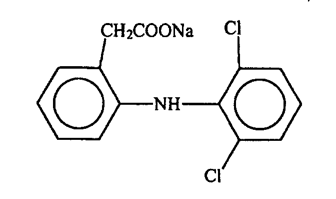 sind bekannte und gut eingeführte, nicht steroidale Antirheumatika, die sowohl oral als auch parenteral, nämlich in Form von Präparaten zur i.m. Injektion, breite Anwendung gefunden haben.

Ein Nachteil dieses Wirkstoffes ist allerdings, dass geringe Mengen in wässriger Lösung bei erhöhter Temperatur unter den Bedingungen der Hitzesterilisation zu 1-(2,6-Dichlorphenyl)-indolin-2-on unter Abspaltung von Hydroxidionen cyclisieren können. Durch Oxidation mit in der Lösung vorhandenem Sauerstoff können sich dann weitere unerwünschte Folgeprodukte bilden (EP 0595.766, Ciba Geigy).

Vor allem für die parenterale Verabreichung wurden daher Präparate entwickelt, die eine relativ hohe Konzentration an Wirkstoff (75mg Diclofenac-Na auf ein Volumen von 2-3ml) besitzen, die dann intramuskulär verabreicht werden. Diese Präparate für die i.m. Gabe benötigen dabei Wasser als Verdünnungsmittel, dem jedoch eine hohe Konzentration an Lösungsvermittlern, nämlich gemäß EP 0595.766 zum Beispiel 5-50%, vorzugsweise 20 bis 35% Gew./Vol. zugesetzt werden muß.

Als solche Lösungsvermittler werden gemäß EP 0595.766 1,2-Propylenglykol oder Polyethylenglykol mit einer Molmasse von ca. 300 bis 400 eingesetzt.

Weiters werden gemäß dieser Druckschrift außerdem Stabilisatoren hinzugefügt, die pH-Stabilität herbeiführen und dadurch unerwünschte Cyclisierung verhindern sollen. Als solche Stabilisatoren sind u. a. Glutathion oder N-Acetylcystein empfohlen, die aber beide in Kombination mit anderen Substanzen verwendet werden. So soll z.B. N-Acetyl-cystein entweder zusammen mit Glutathion oder mit Alkancarbonsäurealkylester oder Hydroxyalkancarbonsäurealkylester eingesetzt werden, wobei die Säuren 2 bis 3 C-Atome bzw. 2 bis 4 C-Atome im Alkylrest aufweisen und bei Säuren mit oder ohne Hydroxygruppe der Alkylrest der Estergruppierung 2 bis 3 C-Atome besitzt.

Bevorzugt ist dabei eine Kombination von Milchsäureäthylester mit Glutathion oder N-Acetylcystein.

Wenn in dieser Patentanmeldung neben einer intramuskulären Anwendung der beschriebenen Präparate auch von einer möglichen i.v. Verabreichung dieser konzentrierten Präparate gesprochen wird, so ist festzustellen, dass sich in der Praxis die i.v. Anwendung so hoch konzentrierter Präparate nicht nur nicht durchgesetzt hat, sondern sogar ausdrücklich abgelehnt wird. So ist z. B. für das Injektionspräparat auf Basis Diclofenac-Na Diclobene ®-Ampullen 75mg (2ml), das in Österreich zugelassen ist, in den Publikationen der Österr. Apotheker Verlagsgesellschaft ausdrücklich festgehalten, daß diese Ampullen für eine Bolusinjektion nicht verwendet werden dürfen.

Für besonders akute Fälle wurde es jedoch als wünschenswert empfunden, das Diclofenac-Na in wesentlich verdünnterer Lösung als Infusion verabreichen zu können. Aus diesem Grunde wurden für Infusionen die für die i.m.-Verabreichung hergestellten Ampullen mit 2 bis 3ml Volumen und einem Wirkstoffgehalt von 75mg pro Ampulle für eine Infusion mit 100, 125 oder 250ml physiologischer Kochsalzlösung, 5%-iger Glucoselösung oder Laevuloselösung versetzt. Diese Infusionslösungen wurden dann unmittelbar nach der Verdünnung verabreicht. Dabei haben sich Infusionslösungen, die durch Zusatz von 250ml physiologischer Kochsalzlösung auf eine Ampulle erhalten wurden, am besten bewährt, da bei dieser Verdünnung am wenigsten unerwünschte Nebenwirkungen beobachtet worden sind. (R. Eberl und H. Bröll, Therapiewoche Österreich 4 (1989) 429-436). Diese Infusionslösungen sollen gemäß den oben erwähnten Angaben zu dem Präparat Diclobene ® Ampullen 75mg unmittelbar nach der Zubereitung in klarem, einwandfreien Zustand über 30-60 Minuten infundiert werden.

Da die Verdünnung an Ort und Stelle Risken mit sich bringt, wurde bald der Wunsch nach einer Fertiginfusion geäußert, die trotz der hohen Verdünnung eine ausreichende Stabilität bei Umgebungstemperatur über eine Lagerdauer von mindestens 12 Monaten besitzen sollte.

Dies gelang mit der Schaffung der Fertiginfusion Neodolpasse ®, deren Anwendung von Uitz E., Aglas F., Wurm A., Rainer F., in Wiener Medizinische Wochenschrift 1998, 148,179-182 beschrieben ist. Diese Infusion enthält jedoch neben dem Diclofenac-Na einen weiteren Wirkstoff, nämlich Orphenadrin-citrat, das muskelrelaxierend wirkt. Die genaue Zusammensetzung dieser Lösung, die sich gemäß Uitz et al. sehr bewährt hat, lautet 0,3 g/l Diclofenac-Na, 0,120 g/l Orphenadrin-citrat, 1,00 g/l N-Acetylcystein, 0,10 g/l Na-EDTA. 2 H₂O, 12,7 g/l L-Äpfelsäure, 1n NaOH bis pH 7,5-7,7 und Wasser ad inj. ad 1000 ml. Diese Infusionslösung, die sich durch gute Lanzeitstabilität auszeichnet, enthält also kein organisches Lösungsmittel als Lösungsvermittler und N-Acetylcystein als alleiningen Stabilisator. Die Stabilität der Lösung wird u. a. auch durch die Gegenwart der Äpfelsäure beeinflußt, die nicht nur eine Pufferwirkung entfaltet, sondern, was wichtig ist, als Natriumreserve für das Diclofenacsalz dient, da sonst das Diclofenac durch das saure Orphenadrincitrat zum Teil in freies Diclofenac umgewandelt werden könnte. Darüber hinaus wird mit der Äpfelsäure auch die Osmolarität eingestellt.

Im Rahmen der Anwendung dieser Infusionslösung zeigte es sich allerdings, dass der Orphenadringehalt nicht immer erwünscht ist, und sich daher ein Bedarf nach einer Infusionlösung mit einem Diclofenac-Na-Gehalt als alleinigen Wirkstoff ergab, wobei die Konzentration des Diclofenac-Na etwa im gleichen Bereich liegen sollte, wie beim Neodolpasse ®. Die Erfindung stellt sich nun die Aufgabe, eine solche Infusionslösung bereitzustellen.

Bei den Versuchen zur Herstellung einer solchen Infusionslösung zeigte es sich überraschenderweise, dass es möglich ist, eine Infusionslösung mit einem Gehalt an Diclofenac-Na in etwa der Menge entsprechend dem Gehalt an Diclofenac-Na der oben geschilderten Infusionslösung zu schaffen, die neben N-Acetylcystein und Na-EDTA nur Kochsalz oder andere physiologische Substanzen zur Einstellung der Osmolarität und Wasser enthält. Das bedeutet, dass auf eine Pufferung zur Erzielung einer pH-Stabilität verzichtet werden konnte.

Nach Einstellung des pH-Wertes von über 7,5 und einer thermischen Behandlung vorzugsweise von 30 Minuten bei 80°C, die keine wesentliche pH-Änderung mit sich bringt, bleibt bei der erfindungsgemäßen Lösung der pH-Wert bei Lagerung zunächst in diesem Bereich. Bei Lagerung über mehrere Monate macht sich ein Anstieg des pH-Wertes der Lösung bemerkbar, der auf eine beginnende Cyclisierung zurückzuführen ist. Überraschenderweise ist dieser Anstieg jedoch trotz der hohen Verdünnung sehr langsam, sodass dieser Anstieg des pH-Wertes erst nach Monaten pH-Werte erreicht, die das Ende der Lagerzeit markieren. In der Regel und unter Einhaltung bestimmter Bedingungen handelt es sich um 1 ½ Jahre, bis dieser Punkt erreicht ist, was erstaunlich ist, da nur N-Acetyl-L-cystein als Stabilisator eingesetzt wird. Wie überraschend das ist, zeigt die EP 0595.766 für konzentrierte Lösungen von Diclofenac-Na, die mit Acetyl-cystein in Kombination mit Milchsäureäthylester stabilisiert wurden, bei denen eine Stabilität über "mehrere Monate" und nicht, wie erfindungsgemäß über 1 Jahr, als wesentlicher Erfolg dargestellt wurde.

Gegenstand der vorliegenden Erfindung ist demnach eine lagerstabile wässrige Infusionslösung von Diclofenac-Salzen in therapieoptimierter Konzentration, bestehend aus einem i.v. verträglichen Diclofenac-Salz in einer Menge entsprechend 200-600mg/l Diclofenac-Natrium, 100-1000mg/l N-Acetyl-cystein als Stabilisator, 50-100 mg/l Natrium-EDTA.2H₂O, sowie eine physiologische Substanz zur Einstellung der Osmolarität auf physiologische Werte, deren pH-Wert 7,6-9,0 beträgt.

Bevorzugtes Diclofenac-Salz ist Diclofenac-Na. Es können aber auch das K-Salz des Diclofenacs oder i.v. verträgliche Salze von physiologisch unbedenklichen Aminen wie z.B. Mono-, Di- oder Tri-C₍₁₋₄₎-alkylamine in entsprechender Menge eingesetzt werden.

Die Menge an Diclofenac-Natrium beträgt vorzugsweise 400mg/l.

Ferner ist es empfehlenswert, die Infusionslösung mit 1000mg/l N-Acetyl-L-cystein zu stabilisieren und die Menge an Natrium-EDTA.2H₂O mit 100mg/ml zu bemessen.

Als Mittel zur Isotonisierung sind vor allem Natriumchlorid und Glycerin zu nennen, es können aber auch Zuckeralkohole, wie Mannit, eingesetzt werden.

Bei einer derart verdünnten wäßrigen Lösung, wie es die erfindungsgemäße Lösung ist, wäre es zu erwarten gewesen, dass relativ schnell Hydrolysen verbunden mit einem Absinken des pH-Wertes eintritt, was einen negativen Einfluß auf die Stabilität der Lösung mit sich bringen würde. Wie schon oben erwähnt, ist das Gegenteil der Fall. Dabei kann es toleriert werden, dass der pH-Wert den für die Herstellung definierten oberen Grenzwert von 8,1 bei der Lagerung überschreitet. Es hat sich gezeigt, dass ein Anstieg des pH-Wertes bis auf 9 als unbedenklich einzustufen ist, da die erfindungsgemäße Lösung in Abwesenheit eines Puffersystems keine Pufferkapazität besitzt. Beim Verabreichen der Lösung wird diese vom Blut sofort "neutralisiert". Das Ende der Lagerstabilität der Lösung wird durch das Überschreiten eines pH-Wertes von 9 definiert. Diese Stabilitätsphase der Lösung überschreitet üblicherweise 1 Jahr und erreicht häufig 18 Monate. Die Verunreinigung mit dem dabei entstandenen Laktam bleibt gering. Sie beträgt bezogen auf die Gesamtlösung Werte im einstelligen ppm-Bereich.
Die erfindungsgemäße Infusionslösung zeichnet sich durch eine sehr gute Verträglichkeit aus, wobei es sogar möglich geworden ist, den Wirkstoffgehalt von dem bisherigen Maximum von 300mg/l bis auf 600mg/l zu steigern. Besonders bevorzugt ist eine Infusionslösung mit einem Gehalt an Diclofenac-Na von 400mg/l. Die Erhöhung des Wirkstoffgehalts führt dabei überraschenderweise zu keinen Einbußen an der Verträglichkeit.

Die erfindungsgemäße Lösung wird folgendermaßen hergestellt:

Für die Herstellung der erfindungsgemäßen Infusionslösung ist es wesentlich, dass der Ansatz auf einen pH-Wert von 7,6-8,1; vorzugsweise auf 7,8, eingestellt wird. Ein Unterschreiten der pH-Grenze von 7,6 führt zu einer Beeinträchtigung der Stabilität bereits in der Anfangsphase der Lagerung. Die besten Ergebnisse werden bei einem Ausgangs-pH von 7,8 erreicht. Die Obergrenze von pH 8,1 ist zu wählen, um die Lagerzeit der Lösung nicht unnötig zu verkürzen.

Zur Bereitung der erfindungsgemäßen Lösung wird unter Stickstoffatmosphäre leicht angewärmtes Wasser (ca. 25 - 40°C) ad inj. vorgelegt, dem in der Folge zunächst das N-Acetyl-cystein, dann das Natrium-EDTA .2H₂O und weiter die zur Einstellung der Osmolarität nötige Menge an physiologischer Substanz jeweils eingetragen und aufgelöst wird. Die resultierende Lösung wird daraufhin durch Zusatz einer physiologisch verträglichen Base, vorzugsweise Natronlauge, auf einen pH-Wert im Bereich von 7,6-8,1 eingestellt, worauf dann das Diclofenac-Salz eingetragen und aufgelöst wird und die Lösung nach gegebenenfalls nötiger Feineinstellung des pH-Werts, Auffüllen auf das entsprechende Endvolumen und aseptischer Abfüllung einer abschließenden thermischen Behandlung bei einer Temperatur von höchstens 90°C, vorzugsweise 30 Minuten bei 80°C, unterzogen wird.

Die erfindungsgemäße Infusionslösung ist vor allem zur Verwendung als Fertiginfusion bei akuten Schmerzzuständen vorgesehen. Dabei handelt es sich sowohl um rheumatische, als auch nichtrheumatische Krankheitsgeschehen verbunden mit starken Schmerzen und/oder Entzündungen, wie rheumatische Arthritis, Osteoarthritis, sowie Fällen von Spondylitis. Es können aber auch andere Schmerzzustände wie Nierenkoliken, posttraumatische oder postoperative Zustände sowie Schmerzen nach zahnärztlichen Eingriffen behandelt werden. Die Konzentration von 200-600mg/l Diclofenac-Natrium, insbesondere jene von 400mg/l, ist therapieoptimiert. Die besonders bewährte Dosis sind 100mg Wirkstoff in 250ml Einheiten, die in der Regel als tägliche Dosis eingesetzt werden.

Beispiel 1: 800ml Wasser ad inj. werden in einem Rührgefäß auf 25°C erwärmt und 15 Minuten mit Stickstoff begast. In dieses vorgelegte Wasser werden 1g N-Acetyl-L-cystein eingetragen und aufgelöst, ferner 0,1g Natrium-EDTA.2H₂O und schließlich 8,4g Natriumchlorid hinzugefügt und aufgelöst, worauf die Lösung durch Zugabe von 0,3g Natriumhydroxid auf einen pH-Wert von 7,8 voreingestellt und anschließend mit 0,4g Diclofenac-Natrium versetzt wird. Nach erfolgter Auflösung unter Stickstoffbegasung wird mit 1n NaOH/1nHCl der pH-Wert falls nötig auf 7,8 korrigiert, das Volumen mit Wasser ad inj. auf 11 aufgefüllt wird, worauf die Lösung unter konstanter Stickstoffbegasung über ein Sterilfilter (0,2µm Porenweite) unter Laminar Air Flow in vier 250ml Flaschen abgefüllt wird. Sie werden dann 30 Minuten bei 80°C im Autoklaven thermisch behandelt.

Der pH-Wert nach Autoklavierung betrug 7,85-7,9. Das Präparat erreichte nach 18 Monaten die Haltbarkeitsgrenze von pH 9,0.
Beispiel 2: 800ml Wasser ad inj. werden wie in Beispiel 1 beschrieben nach Stickstoffbegasung auf 35°C erwärmt und aufeinanderfolgend mit 0,1g N-Acetyl-L-cystein und 0,1g Natrium-EDTA.2H₂O versetzt und zur Lösung gebracht. Zur Isotonisierung werden 27g Glycerin hinzugefügt und eingemischt, worauf der pH-Wert durch Zufügen von 1n Natronlauge auf pH 7,75 eingestellt wurde. In dieser Lösung wurden dann wie in Beispiel 1 beschrieben 0,4g Diclofenac-Natrium aufgelöst und das Volumen mit Wasser ad inj. auf 1000ml aufgefüllt.

Die resultierende Lösung wurde wie in Beispiel 1 in 4 Portionen abgefüllt und autoklaviert, und ist damit als Infusionslösung für die Anwendung zur Schmerzbekämpfung bereit.

## Patentansprüche

1. Lagerstabile wässrige Infusionslösung von Diclofenac-Salzen in therapieoptimierter Konzentration, bestehend aus einem i.v. verträglichen Diclofenac-Salz in einer Menge entsprechend 200-600mg/l Diclofenac-Natrium, 100-1000mg/l N-Acetyl-L-cystein als Stabilisator, 50-100mg/l Na-EDTA.2H₂O sowie einer physiologischen Substanz zur Einstellung der Osmolarität auf physiologische Werte, deren pH-Wert 7,6-9,0 beträgt.

2. Stabile Infusionslösung gemäß Anspruch 1, **dadurch gekennzeichnet, dass** das Diclofenac-Salz Diclofenac-Na ist.

3. Stabile Infusionslösung gemäß Anspruch 2, **dadurch gekennzeichnet, dass** die Menge an Diclofenac-Natrium 400mg/l beträgt.

4. Stabile Infusionslösung gemäß den Ansprüchen 1-3, enthaltend 400mg/l Diclofenac-Natrium, 1000mg/l N-Acetyl-L-cystein, 100mg/l Natrium-EDTA.2H₂O, und als Mittel zur Isotonisierung Natriumchlorid, Glycerin und/oder Mannit.

5. Verfahren zur Herstellung der lagerstabilen Infusionslösung gemäß den Ansprüchen 1-4, **dadurch gekennzeichnet, dass** in vorgelegtes, leicht angewärmtes Wasser für Injektionszwecke unter Stickstoffatmosphäre N-Acetyl-L-cystein, Natrium-EDTA.2H₂O sowie die zur Isotonisierung erforderliche Menge an physiologischer Substanz eingetragen und aufgelöst werden, die resultierende Lösung durch Zusatz einer physiologisch verträglichen, wasserlöslichen Base auf einen pH-Wert im Bereich von 7,6-8,1 eingestellt wird, das Diclofenac-Salz eingetragen und die Lösung, nach gegebenenfalls nötiger Feineinstellung des pH-Werts, Auffüllen auf das Endvolumen mit Wasser ad inj. und aseptischer Abfüllung einer abschließenden thermischen Behandlung bei höchstens 90°C unterworfen wird.

6. Verfahren nach Anspruch 5, dass als physiologisch verträgliche, wasserlösliche Base Natronlauge eingesetzt wird.

7. Verfahren nach den Ansprüchen 5 und 6, **dadurch gekennzeichnet, dass** die Lösung nach Zusatz des Diclofenacsalzes auf pH 7,8 eingestellt wird.

8. Verfahren nach den Ansprüchen 5-7, **dadurch gekennzeichnet, dass** die thermische Behandlung 30 Minuten bei 80°C durchgeführt wird.

9. Lagerstabile Infusionslösung von Diclofenac-Salzen gemäß den Ansprüchen 1 bis 4, zur Verwendung als Fertiginfusion bei der Behandlung von insbesondere akuten Schmerzzuständen.

## Claims

1. A shelf stable aqueous infusion solution of salts of diclofenac in a concentration optimised for therapy, consisting of a salt of diclofenac that is intravenously compatible in an amount corresponding to 200 to 600 mg/l diclofenac sodium, 100 to 1000 mg/l N-acetyl-L-cysteine as stabilizer, 50 to 100 mg/l Na-EDTA.2H₂O, as well as a physiological substance for the adjustment of the osmolarity to physiological values, of which the pH value is 7.6 to 9.0.

2. A stable infusion solution according to claim 1, **characterized in that** the salt of diclofenac is diclofenac sodium.

3. A stable infusion solution according to claim 2, **characterized in that** the amount of diclofenac sodium is 400 mg/l.

4. A stable infusion solution according to claims 1 to 3, containing 400 mg/l diclofenac sodium, 1000 mg/l N-acetyl-L-cysteine, 100 mg/l sodium EDTA.2H₂O and sodium chloride, glycerol and/or mannite as a mean for isotoisation.

5. A process for the production of the shelf stable infusion solution according to claims 1 to 4, **characterized in** bringing in and dissolving in provided, slightly warmed water for injection purpose under nitrogen atmosphere N-acetyl-L-cysteine, sodium EDTA.2H₂O, as well as the amount necessary for isotonisation of the physiological substance, adjusting the pH value of the resulting solution to a range between 7.6 to 8.1 by adding a physiologically compatible water soluble base, bringing in the diclofenac salt, and, after optionally necessary fine adjustment of the pH value, filling to the end volume with water for injection purpose, aseptic filling, and, subsequently applying a heat treatment at temperatures not exceeding 90 °C.

6. A process according to claim 5, **characterized in that** as physiologically compatible water soluble base sodium hydroxide is applied.

7. A process according to claims 5 and 6, **characterized in that** after the addition of the salt of diclofenac the solution is adjusted to a pH value of 7.8.

8. A process according to claims 5 to 7, **characterized in that** the heat treatment is conducted at 80 °C for 30 minutes.

9. A shelf stable infusion solution of salts of diclofenac according to claims 1 to 4 for the use as instant infusion particularly for the treatment of acute states of pain.

## Revendications

1. Solution aqueuse pour perfusion stable à l'entreposage de sels de diclofenac en une concentration optimisée en fonction de la thérapie, constituée par un sel de diclofenac acceptable pour une administration par voie intraveineuse en une quantité correspondant à 200-600 mg/l de diclofenac-sodium, par 100-1000 mg/l de N-acétyl-L-cystéine à titre de stabilisateur, par 50-100 mg/l de Na-EDTA.2H₂O, et par une substance physiologique pour le réglage de l'osmolarité à des valeurs physiologiques, dont la valeur de pH s'élève de 7,6 à 9,0.

2. Solution stable pour perfusion selon la revendication 1, **caractérisé en ce que** le sel de diclofenac est du diclofenac-Na.

3. Solution stable pour perfusion selon revendication 2, **caractérisé en ce que** la quantité du diclofenac-Na s'élève à 400 mg/l.

4. Solution stable pour perfusion selon les revendications 1 à 3, contenant 400 mg/l de diclofenac-Na, 1000 mg/l de N-acétyl-L-cystéine, 100 mg/l de Na-EDTA.2H₂O et, à titre d'agent pour l'isotonisation, du chlorure de sodium, du glycérol et/ou du mannitol.

5. Procédé pour préparer la solution pour perfusion stable à l'entreposage selon les revendications 1 à 4, **caractérisé en ce que**, dans de l'eau tiédie à des fins d'injection déposée au préalable, sous atmosphère d'azote, on introduit de la N-acétyl-L-cystéine, du Na-EDTA.2H₂O, ainsi que la quantité requise pour l'isotonisation d'une substance physiologique ; on procède ensuite à une dissolution ; on règle la solution résultante à une valeur de pH dans la plage de 7,6-8,1 par addition d'une base hydrosoluble physiologiquement acceptable ; on introduit le sel de diclofenac et on soumet la solution, après un réglage fin de la valeur de pH éventuellement requis, remplissage pour obtenir le volume final avec de l'eau pour injection et transvasement aseptique, à un traitement thermique ultérieur à une température maximale de 90 °C.

6. Procédé selon la revendication 5, **caractérisé en ce qu'**on met en oeuvre, à titre de base hydrosoluble physiologiquement acceptable, de la lessive de soude.

7. Procédé selon les revendications 5 et 6, **caractérisé en ce qu'**on règle la solution à un pH de 7,8 après addition du sel de diclofenac.

8. Procédé selon les revendications 5 à 7, **caractérisé en ce qu'**on effectue le traitement thermique pendant 30 minutes à 80 °C.

9. Solution pour perfusion de sels de diclofenac stable à l'entreposage selon les revendications 1 à 4, à des fins d'utilisation à titre de perfusion prête à l'emploi lors du traitement d'états de douleurs en particulier aigus.
